# EUROPEAN PATENT SPECIFICATION

(11) **EP 0 238 190 B1**
(45) Date of publication and mention of the grant of the patent: **21.07.1993**
(21) Application number: 87301163.9
(22) Date of filing: 11.02.1987
(51) Int. Cl.: G01N 1/28, G01N 33/574, G01N 33/577

(54) **Multi-tumour tissue slices, methods for their production and testing the tissues in such slices**
Vieltumorige Gewebescheiben, Verfahren zu deren Herstellung und Untersuchung der Gewebe in solchen Scheiben
Tranches minces de tissu de multi-tumeur, leurs procédés de fabrication et essai du tissu dans ces tranches

(30) Priority: 12.02.1986 US 828775
(43) Date of publication of application: 23.09.1987
(73) Proprietor: Beckman Research Institute of the City of Hope, Duarte California 91010 (US)
(72) Inventor: Battifora, Hector A., Arcadia California 91006, (US)
(74) Representative: De Minvielle-Devaux, Ian Benedict Peter

(56) References cited:
- EP-A- 0 117 262
- EP-A- 0 139 424
- EP-A- 0 159 603
- BE-A- 731 769
- US-A- 2 964 443
- JOURNAL OF IMMUNOLOGICAL METHODS vol. 86, no. 1, 1986, pages 17-20, Amsterdam, Netherlands; A. IMAM: "A novel immunoperoxidase procedure of using human monoclonal antibodies for the detection of cellular antigens in tissue sections"

## Description

This invention relates to multi-tumor tissue blocks and to methods of producing and using such blocks. The invention particularly relates to methods of producing a plurality of slides each containing a plurality of tissues with substantially identical characteristics to those of corresponding tissues in the other slides. In this way, all of the tissues in a slide can be tested with a single drop of a material such as a hybridoma supernatant.

In recent years, rapid progress has been made in applying immunohistochemical method to solving problems in histopathologic diagnosis. This progress has been largely stimulated by the development of hybridoma techniques for producing monoclonal antibodies. However, techniques for screening and applying such antibody have not kept pace with the techniques of producing such antibodies. Among the numerous new monoclonal antibodies, the identification of those clinically useful requires their specificity and sensitivity to be established by laborious immunohistological studies involving a large number of normal tissues and neoplasms. These studies have been generally performed on individual slides each of which contains a section from a single specimen.

Because of the use of slides with single tissue samples (or slices), the clinical studies now being performed have certain significant disadvantages. For example, the studies are slow and expensive because numerous slides have to be individually prepared and processed. The studies are also inherently limited in accuracy because each individual tissue section is not similar to the other slides. Limitations in accuracy also result because each slide cannot be processed in the same manner as the other slides.

A substantial effort has been made in recent years to provide methods and slides which will eliminate the disadvantages and limitations discussed above. In spite of such efforts and the considerable costs involved in such efforts, such disadvantages and limitations still persist.

In one embodiment of the invention, a plurality of tissue rods which have a relatively great length such as ten millimeters (10 mm) and a relatively small cross-sectional area such as one square millimeter (1 mm²) in a transverse, preferably perpendicular, plane. The rods may constitute (1) an array of well characterized neoplasms of varying histogenesis and degrees of differentiation and a spectrum of normal tissues, (2) groups of tissues with related types of tumors and/or normal tissues, (3) tissue groups of defined classes and/or tissue groups as control samples and/or (4) representative tissue samples from defined patient populations.

The rods are disposed in substantially parallel relationship in a casing which may have dimensions of approximately two centimeters (2 cm) by three centimeters (3 cm). The casing may be formed from a suitable material such as portions of small intestines of small animals such as rabbits and may be stretched, preserved and stored. The casing may be tightly wrapped around the rods and bound as by a fine thread. The ends of the rods and the casing may be trimmed to insure that the rod ends are exposed. When the rods are disposed in groups in the casing, septums in the casing may separate the different groups.

The thread is removed and the combination is embedded as in paraffin. The paraffin block is then sectioned to produce as many as one thousand (1000) substantially identical slides. A single drop of a suitable material such as a hybridoma supernatant may then be applied to individual ones of the slides to react with the sectioned rods in the slides. The staining intensity of each rod section indicates the relative amount of reaction between the hybridoma supernatant and such rod section.

In the drawings:
Figure 1 is a schematic perspective view of a tissue rod which is included in one embodiment of the invention;
Figure 2 is a schematic perspective view illustrating a casing having a flat disposition and further illustrates the parallel disposition on the casing of a plurality of tissue rods corresponding to the tissue rod shown in Figure 1;
Figure 3 is a schematic perspective view of the arrangement shown in Figure 2 after the casing has been tightly wrapped and a thread has been wound around the tightly wrapped casing and the thread has been tied;
Figure 4 is a perspective view of the arrangement shown in Figure 3 after the ends of the casing have been trimmed to expose the ends of the tissue rods within the casing;
Figure 5 is a perspective view of the trimmed arrangement shown in Figure 4 after the thread has been removed and the arrangement has been embedded in a suitable material such as paraffin;
Figure 6 is a perspective view of slices of the embedded arrangement shown in Figure 5;
Figure 7 is an elevational view of one of the slices shown in Figure 6 and schematically illustrates the casing and the tissue rods within the casing;
Figure 8 is a schematic perspective view illustrating the application of a drop of a suitable material to be tested such as a hybridoma supernatant or an antiserum or other tissue reagent to the surface of a slice shown in Figures 6 and 7 to stain the exposed ends of the tissue rods in the slice;
Figure 9 is an elevational view schematically illustrating the staining of the exposed surfaces of individual ones of the tissue rods in the slice of Figures 6, 7 and 8 in accordance with the application of the hybridoma supernatant to the slice;
Figure 10 is an elevational view of a slice and schematically illustrates the disposition of a septum in the casing to separate the area within the casing into two (2) separate compartments; and
Figures 11 through 16 show slices actually obtained by applicant by practicing the invention as shown in Figures 1 through 10.

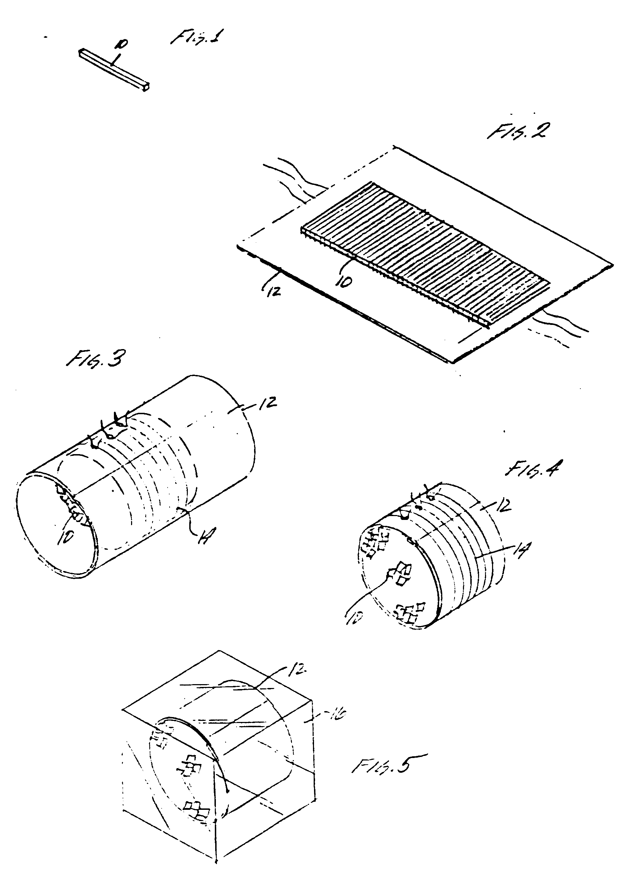

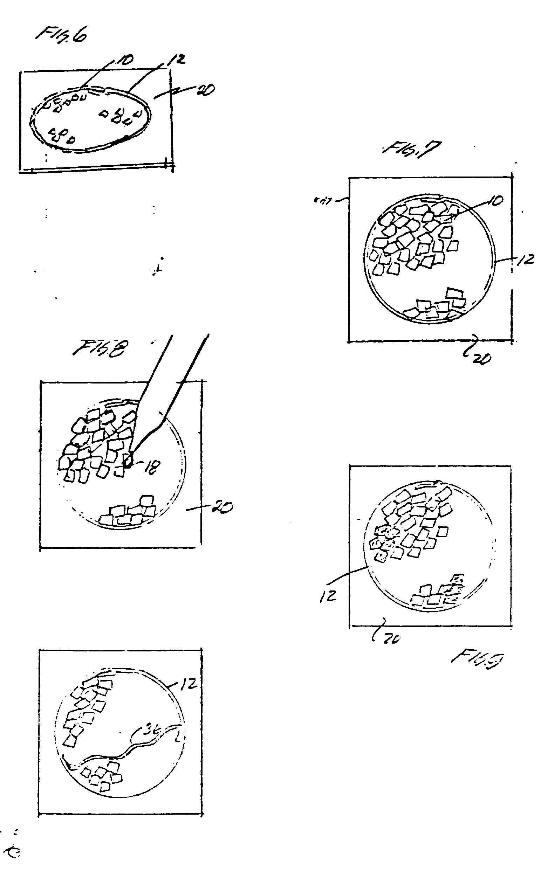

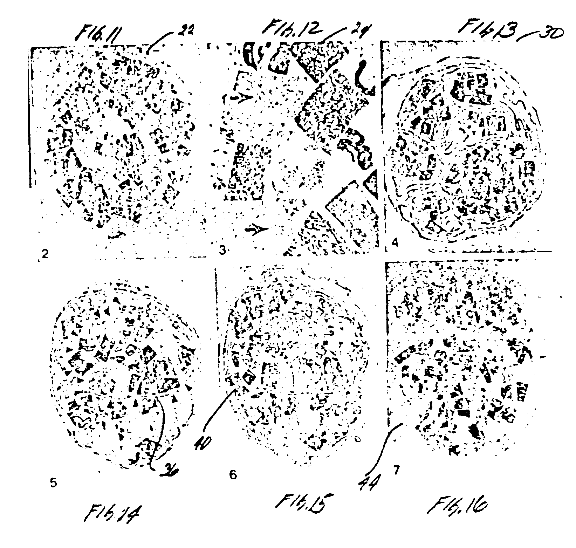

In one embodiment, a plurality of tissues 10 are provided. Each of the tissues may constitute rods having a suitable length such as approximately ten millimeters (10 mm) and a suitable cross-sectional area such as approximately one square millimeter (1 mm²) in a plane transverse, preferably substantially perpendicular, to its length.

Each of the tissues may have been previously embedded in a material such as paraffin. The tissues are removed from the paraffin blocks in a conventional manner and deparaffinized in a material such as xylene. The tissues may then be rehydrated as in ethanol at a graded series of concentrations to a final concentration such as approximately fifty per cent (50%). The rehydrated tissue is then sliced as with a sharp razor blade into the sections which have a suitable thickness such as approximately one millimeter (1 mm). These sections are further divided as by a razor blade into the tissue rods 10 having approximately one square millimeter (1 mm²) in cross section.

As many as one hundred (100) or more of the tissue rods 10 may be stacked in parallel on a casing 12. Preferably the tissue rods 10 are disposed in closely stacked relationship on the casing 12. The casing 12 may be prepared from a suitable material such as a portion of the small intestine of a small mammal such as a rabbit. Before the tissue rods are disposed in the casing 12, the material of the casing 12 is opened, stretched as over a cork board, preserved or fixed as in formalin and stored, until needed, as in a solution containing fifty per cent (50%) of ethanol. The casing 12 may have suitable dimensions such as approximately two centimeters (2 cm) by three centimeters (3 cm).

After the tissue rods 10 have been disposed in a closely stacked and substantially parallel relationship on the casing 12, the casing 12 is tightly wrapped around the tissue rods 10 and is maintained in this tightly confining relationship as by a fine thread 14. The ends of the resultant cylindrical package are subsequently trimmed as by a razor blade to expose the ends of all the rods at the exposed ends of the casing 12.

The fine thread 14 may thereafter be removed from the periphery of the casing 12. The cylindrical package containing the tissue rods 10 are subsequently embedded in a conventional manner into a block 16 made from a suitable material such as paraffin such that the ends of the tissue rods are substantially perpendicular to the face of the block 14 and are exposed at the face of the block. The paraffin block 16 may thereafter be sliced to form slices 18. The slicing is in a plane substantially perpendicular to the length of the rods 10. In this way, one thousand or more slices 20 can be produced from each paraffin block 16.

A suitable reagent such as a hybridoma supernatant 18 (Figure 8) or an antiserum or other reagents may be applied to the surface of the slices 20. Since the tissue rods 10 are closely spaced on the surface of each slice 20, only a drop of the reagent has to be applied to each slice. The reagents react with the tissue rods 10 and its presence may be detected with the application of further reagents to stain the exposed surfaces of the rods. The relative degree of such staining indicates the relative amount of reaction between the exposed surface of the tissue rods 10 and the reagent.

The tissue rods 10 may be disposed in different modes in the slice 20. In one mode, a broad array of tissue rods 10 constituting well characterized neoplasms of varying histogenesis and degree of differentiation may be provided in the slices 20. A wide spectrum of normal tissues may also be disposed in such slices. Typically, adenocarcinomas of various origins and squamous cell, undifferentiated and neuroendocrine carcinomas, lymphomas, melanomas, assorted sarcomas and samples of uncommon neoplasms may be disposed in the slices 20. These slices are particularly advantageous for for screening monoclonal antibodies in the early stages of hybridoma preparation. Tissue or tumor specificity, and any unexpected reactivity of these, can readily detected when a single drop of hybridoma supernatant is applied to these slices.

Figure 11 shows a slice 22 (magnified eight (8) times) which contains a broad variety of samples of neoplasms. This slice has been immunostained in a conventional manner by the ABC method with a drop of a hybridoma supernatant obtained from spleen lymphocytes of a mouse injected with human pancreatic carcinoma cells. After being stained by the hybridoma supernatant, the slice 22 was counterstained with hematoxylin. Only four (4) tissues (all gastrointestinal adenocarcinomas) show intense immunostaining . This indicates that a monoclonal antibody with some specificity is present in the supernatant.

Slices such as the slices 22 in Figure 11 have been used for immunohistochemical studies with many different antibodies. In such slices, tissues with several degrees of differentiation and with variable density of antigen expression have been provided within the slices. It has been possible to control the sensitivity of each such procedure and to monitor if there are any daily variations in the immunostaining method. Neoplasms with low antigen levels can be identified in each slice by their low staining intensity. If these neoplasms fail to stain the tissue rods in any particular slice, they provide an indication that the sensitivity of the procedure has decreased. Appropriate corrective measures can then be implemented.

Figure 12 is a close-up view, with a magnification of ten (10), of a portion of a slice 24 stained with a cocktail of monoclonal antibodies to keratins having a low molecular weight. The field shown in Figure 12 includes samples from fourteen (14) different epithelial neoplasms. The tissues in the slice 24 were stained in a conventional manner by the ABC method and were counterstained with hematoxylin. As will be seen, there is a marked variation in the intensity in which different tissues on the slice 24 are immunostained. Staining is particularly weak in two (2) undifferentiated carcinomas which are indicated by arrows. Tissues of such low antigen density provide a routine sensitivity control.

Slices such as the slices 22 and 24 containing miscellaneous types of tissues may be used for comparative studies of immunohistological methods. In particular, such slices may be used to resolve questions concerning the relative sensitivity of such immunohistological methods. Such slices may also be used as "check samples" in survey studies. For example, such slices may be used to provide inexpensive and reliable comparisons of the results of immunohistological studies among various laboratories.

Since there are a relatively large number of tissues in the slices such as the slices 22 and 24 and since many of these tissues contain similar morphologic features, it may not always be possible to identify the source of each individual tissue in such slices. Any such problem should be minimal, however, since the trained observer can recognize many classes of tumors with little difficulty. Furthermore, any inability to trace each tumor sample to its patient source should not be regarded as a serious impediment to the use of the method constituting this invention because such slices may generally be designed for screening and not for definitive studies.

The tissues 10 may also be disposed in segments such as in the slices 20. These segments may be obtained by wrapping groups of rods from related types of tumors or normal tissues in separate compartments within the casing 12. These compartments may be defined by septums 28 which may be constructed from the same material as the casing 12. This approach is advantageous because it provides for a grouping of related tissues and accordingly provides for an identification of a type of tissue or neoplasm from its position in the slice 20. This allows even technicians with no expertise in the tissue morphology to identify the different types of tissue in the slice and to interpret the results of the antibody screening on the tissues with minimal difficulty.

Figure 13 illustrates a slice 30 (with a magnification of eight (8)), containing seven (7) different compartments or segments. Individual ones of these compartments contain tissues with adenocarcinomas. The slice 30 was stained by the ABC with a heterologous antiserum to protein S100 and was counterstained with hematoxylin. Only the upper compartment shows intense immunostaining. This compartment contains tissues from six (6) different melanomas.

The segmented or compartmentalized slices discussed in the previous paragraph may be theme oriented. These slices are particularly useful for further characterization of antibodies which have shown apparent specificity in screening against tissues in such slices. For example, a slice 32 shown in Figure 14 has been used for comparing monoclonal antibodies specific to prostatic tissue. The slice 32 has been magnified fourteen (14) times.

In Figure 14, a septum 36 divides the tissues in the slice 32 into three (3) compartments. Such a septum is also schematically shown in Figure 10. The upper right compartment in Figure 14 contains nineteen (19) samples of prostatic tissues including well differentiated and poorly differentiated carcinomas. It also contains tissues of normal and hyperplastic prostate. The upper left compartment contains eight (8) samples of neuroendocrine carcinomas of various degrees of differentiation. These carcinomas have been chosen because of their histologic similarity to some prostatic carcinomas. The lower compartment contains twelve (12) samples of non-prostatic adenocarcinomas of various origins. The slice 32 of Figure 14 was obtained by immunostaining the tissues in the slice in a conventional manner by the ABC method and then counterstaining the tissues with hematoxylin. A monoclonal antibody directed against prostatic-specific antigens was used in the immunostaining.

In addition to being used as controls for prostatic markers, the theme-oriented segmented slices may also be used as markers for endocrine differentiation. This is shown in a slice 40 in Figure 15, this slice being shown as being magnified fourteen (14) times. The slice 40 in Figure 15 was obtained from the same paraffin block as the slice 32 in Figure 14. As a result, the tissues in the slice 40 were substantially identical to the tissues in the slice 32 of Figure 14. The tissues in the slice 40 were obtained by the ABC method with a monoclonal antibody to neuron-specific enolase and was counterstained with hematoxylin. Staining was found to be intense only for several of the neuroendocrine carcinomas in the upper left compartment.

Slices may also be formed from tissues disposed in the slices in clinically defined segments. In these slices, representative tissue slices may be included from defined patient populations. For example, a slice 44, magnified seven (7) times in Figure 16, contained approximately ninety (90) tumor samples from two (2) groups of patients with stage I or stage II breast carcinoma. A single septum 46 separated the samples of the patients in one (1) group from the patients in the other group. One group included tissues of stage I and stage II patients who developed metastases or had recurrences in less than two (2) years after mastectomy. The other group was age-matched and stage-matched. The patients in this group were free of disease after a long period of follow-up. The slice was immunostained with a monoclonal antibody prepared against milk fat globule-derived membranes and was lightly counterstained with hematoxylin. Twice as many tumor samples were stained by the monoclonal antibody in the first compartment as in the second compartment. This suggests that the antigen being detected may have prognostic significance.

The tissue slices disclosed above have certain important advantages. One advantage is that errors resulting from procedural variations are effectively eliminated since all of the tissue samples in a slice are treated simultaneously by the same procedure. Quantitative digital readout of immunohistochemistry by microdensitometry may thus be enhanced from a reliability standpoint by the use of slices produced in accordance with this invention. Furthermore, slices out from a single block may be distributed to a number of histopathology laboratories as check samples. These slices may then be of great value as quality control tools in immunohistochemistry.

The small volume in each of the tumors sampled in the slices has not been a serious obstacle to the use of such tumors since these tumor samples have cross sectional areas of approximately one (1) square millimeter (1mm²), this area is sufficiently large to fill a medium-power field of an average light microscope. Many endoscopic-biopsy specimens are not larger than this. Furthermore, sampling errors occur infrequently because the portions of tissue to be employed in the preparation of the slices are selected carefully.

Since only a small amount of hybridoma supernatant is available in the early phase of monoclonal antibody generation, immunohistologic screening is impractical unless a large number of tissues can be grouped within a small surface area. By this procedure, the amount of the hybridoma supernatant required to screen the hybridoma supernatant is minimized. This is accordingly one of the important advantages of this invention. For example, the slices of this invention can probably be used to detect tissue-specific antigens or tumor markers during the early stages of hybridoma preparation.

Although this invention has been disclosed and illustrated with reference to particular embodiments, the principles involved are susceptible for use in numerous other embodiments which will be apparent to persons skilled in the art. The invention is, therefore, to be limited only as indicated by the scope of the appended claims.

## Claims

1. A method of preparing a multi-specimen tissue block comprising:
forming a plurality of tissue specimens containing different marker substances into rods having a relatively small cross-sectional area and a relatively great length;
disposing said rods in a substantially parallel relationship on a casing; and
wrapping said rods in said casing to form a rod bundle.

2. The method of claim 1, further including the step of trimming said rod bundle to expose said specimen rods in transverse cross-section.

3. The method of claim 1 or claim 2, further including the step of embedding said rod bundle in a first embedding medium to form a tissue block.

4. The method of any one of claims 1 to 3, further including the step of slicing said tissue block to provide a plurality of sections, each section comprising a transverse cross-section of each of said tissue specimen rods.

5. The method of claim 4, wherein the tissue block is sliced perpendicular to the longitudinal axis of the rods.

6. The method of any one of claims 1 to 5, wherein each rod has a transverse cross-sectional area of approximately one square millimeter and a length of approximately 10 mm.

7. The method of any one of claims 1 to 6, wherein the casing is prepared from portions of the small intestines of small mammals.

8. The method of any one of claims 1 to 7, wherein, prior to said rods being wrapped in said casing, at least one septum is provided for dividing said plurality of rods into separate groups such that in the wrapped bundle of rods there are at least two groups of rods separated by said septum.

9. The method of claim 8, wherein said at least one septum is formed from said casing.

10. The method of claim 8, wherein said at least one septum is formed by wrapping each separate group of rods individually in a separate casing.

11. The method of any one of claims 8 to 10, wherein the specimen rod in each of said separate groups of specimen rods have properties different from the properties of the specimen rods in the other groups.

12. The method of claim 11, wherein tumor or neoplasm tissues are included in at least one separate group of specimen rods and control specimen rods are included in a different group.

13. The method of claim 11, wherein each separate group of specimen rods includes rods of different tumour tissues.

14. The method of any one of claims 1 to 13, wherein, prior to being formed into rods, the tissue specimens are embedded in a second embedding medium.

15. The method of claim 14, wherein said second embedding medium is removed and the tissue specimens are rehydrated prior to forming the tissue specimens into rods.

16. The method of claim 3 or any claim dependent thereon or claim 14 or any claim dependent thereon, wherein the first or second embedding medium is paraffin.

17. The method of any one of claims 1 to 16, wherein the tissue specimens are marked by different antigenic reactivities.

## Patentansprüche

1. Verfahren zur Herstellung eines mehrere Proben aufweisenden Gewebeblocks, dadurch gekennzeichnet, daß man eine Mehrzahl von Gewebeproben, die verschiedene Marker-Substanzen aufweisen, zu Stäben mit einer verhältnismäßig kleinen Querschnittsfläche und einer verhältnismäßig großen Länge formt
diese Stäbe praktisch parallel zueinander auf einer Umhüllung anordnet und
diese Stäbe in diese Umhüllung unter Bildung eines Bündels von Stäben einwickelt.

2. Verfahren nach Anspruch 1, dadurch gekennzeichnet, daß es weiterhin die Stufe des Zuschneidens dieses Stabbündels umfaßt, um die Probenstäbe im querliegenden Querschnitt freizulegen.

3. Verfahren nach Anspruch 1 oder 2, dadurch gekennzeichnet, daß es weiterhin die Stufe der Einbettung dieses Stabbündels in ein erstes Einbettungsmedium zur Bildung eines Gewebeblocks umfaßt.

4. Verfahren irgendeinem der Ansprüche 1 bis 3, dadurch gekennzeichnet, daß es weiterhin die Stufe des Zerschneidens dieses Gewebeblocks in Scheiben umfaßt, um eine Mehrzahl von Abschnitten zu bilden, wobei jeder Abschnitt in Querrichtung einen Querschnitt jedes dieser Gewebsprobenstäbe aufweist.

5. Verfahren nach Anspruch 4, dadurch gekennzeichnet, daß der Gewebeblock senkrecht zur Achse der Stäbe geschnitten wird.

6. Verfahren nach irgendeinem der Ansprüche 1 bis 5, dadurch gekennzeichnet, daß jeder Stab eine in Querrichtung liegende Querschnittsfläche von etwa einem Quadratmillimeter und eine Länge von etwa 10 mm hat.

7. Verfahren nach irgendeinem der Ansprüche 1 bis 6, dadurch gekennzeichnet, daß die Umhüllung aus Teilen des Dünndarms kleiner Säugetiere hergestellt ist.

8. Verfahren nach irgendeinem der Ansprüche 1 bis 7, dadurch gekennzeichnet, daß vor dem Einhüllen dieser Stäbe in diese Umhüllung wenigstens eine Scheidewand vorgesehen wird, um diese Mehrzahl von Stäben in getrennte Gruppen zu unterteilen, so daß im eingewickelten Bündel von Stäben wenigstens zwei Gruppen von Stäben vorliegen, die durch diese Scheidewand getrennt sind.

9. Verfahren nach Anspruch 8, dadurch gekennzeichnet, daß wenigstens eine Scheidewand aus dieser Umhüllung gebildet wird.

10. Verfahren nach Anspruch 8, dadurch gekennzeichnet, daß wenigstens eine Scheidewand gebildet wird, indem man jede getrennte Gruppe von Stäben einzeln in eine getrennte Umhüllung einwickelt.

11. Verfahren nach irgendeinem der Ansprüche 8 bis 10, dadurch gekennzeichnet, daß die Probenstäbe in jeder dieser getrennten Gruppen von Probenstäben Eigenschaften haben, die verschieden sind von den Eigenschaften der Probenstäbe in den anderen Gruppen.

12. Verfahren nach Anspruch 11, dadurch gekennzeichnet, daß Tumor- oder Neoplasmagewebe in wenigstens eine getrennte Gruppe von Probenstäben einbezogen sind und Kontrollprobenstäbe in einer anderen Gruppe einbezogen sind.

13. Verfahren nach Anspruch 11, dadurch gekennzeichnet, daß jede getrennte Gruppe von Probenstäben Stäbe von verschiedenen Tumorgeweben umfaßt.

14. Verfahren nach irgendeinem der Ansprüche 1 bis 13, dadurch gekennzeichnet, daß vor der Formung zu Stäben die Gewebeproben in ein zweites Einbettungsmedium eingebettet werden.

15. Verfahren nach Anspruch 14, dadurch gekennzeichnet, daß dieses zweite Einbettungsmedium entfernt wird und die Gewebeproben rehydratisiert werden bevor die Gewebeproben zu Stäben geformt werden.

16. Verfahren nach Anspruch 3 oder irgendeinem davon abhängigen Anspruch oder Anspruch 14 oder irgendeinem davon abhängigen Anspruch, dadurch gekennzeichnet, daß das erste oder zweite Einbetttungsmedium Paraffin ist.

17. Verfahren nach irgendeinem der Ansprüche 1 bis 16, dadurch gekennzeichnet, daß die Gewebeproben durch unterschiedliche antigene Reaktivitäten markiert sind.

## Revendications

1. Un procédé de préparation d'un bloc de tissus pour échantillons multiples comprenant:
la formation d'une pluralité d'échantillons de tissus contenant différentes substances de marquage en bâtonnets de section en coupe transversale relativement réduite et de longueur relativement grande;
la disposition de ces bâtonnets en relation sensiblement en parallèle sur une enveloppe ; et
le conditionnement desdits bâtonnets dans ladite enveloppe pour former un faisceau de bâtonnets.

2. Le procédé selon la revendication 1, comprenant l'étape de rognage dudit faisceau de bâtonnets pour exposer lesdits bâtonnets échantillons en coupe transversale.

3. Le procédé selon la revendication 1 ou 2, comprenant, en outre, l'étape consistant à noyer ledit faisceau de bâtonnets dans un premier milieu de noyage pour former un bloc de tissus.

4. Le procédé selon l'une quelconque des revendications 1 à 3, comprenant, en outre, l'étape de tranchage dudit bloc de tissus pour produire une pluralité de tranches, chaque tranche comprenant une coupe transversale de chacun desdits bâtonnets échantillons de tissus.

5. Le procédé selon la revendication 4, dans lequel le bloc de tissus est tranché perpendiculairement à l'axe longitudinal des bâtonnets.

6. Le procédé selon l'une quelconque des revendications 1 à 5, dans lequel chaque bâtonnet a une surface, en coupe droite transversale d'environ un millimètre carré et une longueur d'environ 10 mm.

7. Le procédé selon l'une quelconque des revendications 1 à 6, dans lequel l'enveloppe est préparée à partir de fragments d'intestin grêle de petits mammifères.

8. Le procédé selon l'une quelconque des revendications 1 à 7, dans lequel, avant de conditionner lesdits bâtonnets dans ladite enveloppe, il est prévu, au moins, une cloison pour diviser ladite pluralité de bâtonnets en groupes séparés, de manière à former, dans le faisceau de bâtonnets conditionné, au moins deux groupes de bâtonnets séparés par ladite cloison.

9. Le procédé selon la revendication 8, dans lequel au moins une cloison est ménagée dans ladite enveloppe.

10. Le procédé selon la revendication 8, dans lequel au moins ladite cloison est formée en conditionnant individuellement chaque groupe séparé de bâtonnets dans une enveloppe séparée.

11. Le procédé selon l'une quelconque des revendications 8 à 10, dans lequel les propriétés du bâtonnet échantillon de chacun desdits groupes séparés de bâtonnets échantillons sont différentes des propriétés des bâtonnets échantillons des autres groupes.

12. Le procédé selon la revendication 11, dans lequel des tissus de tumeurs ou de néoplasmes sont inclus dans, au moins, un groupe séparé de bâtonnets échantillons et les bâtonnets échantillons témoins sont inclus dans un groupe différent.

13. Le procédé selon la revendication 11, dans lequel chaque groupe séparé de bâtonnets échantillons comprend des bâtonnets de différents tissus tumoraux.

14. Le procédé selon l'une quelconque des revendications 1 à 13, dans lequel, avant d'être formés en bâtonnets, les échantillons de tissus sont noyés dans un second milieu de noyage.

15. Le procédé selon la revendication 14, dans lequel ledit second milieu de noyage est enlevé et les échantillons de tissus sont réhydratés avant de confectionner les échantillons de tissus en bâtonnets.

16. Le procédé selon la revendication 3 ou toute revendication dépendante de celle-ci ou selon la revendication 14 ou toute revendication dependante de celle-ci, dans lequel le premier ou le second milieu de noyage est de la paraffine.

17. Le procédé selon l'une quelconque des revendications 1 à 16, dans lequel les échantillons de tissus sont marqués par différentes réactivités antigéniques.
